# EUROPÄISCHE PATENTSCHRIFT

(11) **EP 3 864 936 B1**
(45) Veröffentlichungstag und Bekanntmachung des Hinweises auf die Patenterteilung: **08.03.2023**
(21) Anmeldenummer: 19786311.1
(22) Anmeldetag: 08.10.2019
(51) Int. Cl.: H05B 6/64, F24C 7/08

(54) **VERFAHREN ZUM BEHANDELN VON GARGUT UND HAUSHALTS-GARGERÄT**
METHOD FOR TREATING FOOD TO BE COOKED AND DOMESTIC COOKING APPLIANCE
PROCÉDÉ DE TRAITEMENT D'ALIMENTS À CUIRE ET APPAREIL DE CUISSON DOMESTIQUE

(30) Priorität: 09.10.2018 DE 102018217207; 18.10.2018 DE 102018217811
(43) Veröffentlichungstag der Anmeldung: 18.08.2021
(73) Patentinhaber: BSH Hausgeräte GmbH, 81739 München (DE)
(72) Erfinder: EITER, Hans-Martin, 84558 Kirchweidach (DE); SCHÜRF, Stefan, 83313 Siegsdorf (DE)
(86) Internationale Anmeldenummer: PCT/EP2019/077159
(87) Internationale Veröffentlichungsnummer: WO 2020/074474

(56) Entgegenhaltungen:
- EP-A1- 2 618 634
- DE-A1-102017 101 183
- US-A1- 2010 187 224

## Beschreibung

Die Erfindung betrifft ein Verfahren zum Behandeln von Gargut in einem Haushalts-Gargerät mit einem Garraum, mindestens einer Gargutbehandlungseinheit und mindestens einem Sensor zum Bestimmen mindestens einer Oberflächenbeschaffenheit des Garguts, bei dem eine örtliche Verteilung mindestens einer Oberflächenbeschaffenheit des Garguts mittels mindestens eines Sensors gemessen wird. Die Erfindung betrifft auch ein Haushaltsgargerät, aufweisend einen Garraum, mindestens eine Gargutbehandlungseinheit, mindestens einen Sensor zum Bestimmen mindestens einer Oberflächenbeschaffenheit des Garguts, mindestens eine Auswerteeinrichtung und eine Steuereinrichtung zum Ansteuern der mindestens einer Gargutbehandlungseinheit, wobei das Haushaltsgargerät dazu eingerichtet ist, das Verfahren durchzuführen. Die Erfindung ist insbesondere vorteilhaft anwendbar auf ein Haushalts-Gargerät in Form eines Backofens mit oder ohne Zusatzfunktionalitäten, speziell zum Behandeln von Gargut mit einem gleichmäßigen Bräunungsgrad und/oder einer gleichmäßigen Temperaturverteilung an seiner Oberfläche.

DE 10 2014 113 664 A1 offenbart ein Gargerät mit einem Garraum, in dem ein Gargut gegart werden kann, und mit einer Strahlungsquelle, die Strahlung abgeben kann, die zum Garen des Garguts genutzt werden kann. Ferner ist wenigstens ein Sensor vorgesehen, der Parameter des Garguts erfassen kann, insbesondere Glanz, Helligkeit, Position, Kaliber, Bräunung und/oder Bräunungsgrad des Garguts. Zudem ist eine Steuerung vorgesehen, welche zumindest die Leistung der Strahlungsquelle in Abhängigkeit der erfassten Parameter so ansteuert, dass das Gargut durch gezielten Wärmeeintrag in das Gargut und/oder in ein Zubehörteil, auf dem das Gargut angeordnet ist, lokal einen gewünschten Zustand erhält. Ferner ist ein Verfahren zum Garen von Gargut beschrieben.

EP 0 563 698 A2 offenbart ein Kochsystem mit einem Kochbereich mit Stellgliedern, die einstellbar sind, um relevante Parameter zu ändern, die den Kochvorgang eines Nahrungsmittels bestimmen. Eine Kamera überwacht den Kochbereich und steuert einen Prozessor mit Informationen über die Betriebsbedingungen im Kochbereich an. Der Prozessor ist einer Speichervorrichtung zugeordnet, die typische Kochprogramme speichert, die jeweils aus einer unterschiedlichen Kombination von Prozessparametern bestehen. Der Prozessor spricht auf die ankommenden Daten an, um das am besten geeignete unter den gespeicherten Kochprogrammen auszuwählen, wobei er seine Parameter mit der entsprechenden Information von der Kamera vergleicht, um die Stellglieder mit jeweiligen Fehlersignalen zu steuern. Der Kochvorgang wird auf der Grundlage der tatsächlichen Bedingungen des Garguts vollautomatisch geregelt.

DE 10 2014 114 901 A1 offenbart ein Gargerät mit einem Garraum, in dem Gargut gegart werden kann, einem Sensor, der variable Parameter des Garguts und/oder der Umgebung des Garguts erfassen kann, und einer Steuerung, welche die vom Sensor erfassten Signale auswerten kann. Ferner ist ein Verfahren zur Erfassung eines Prozessparameters eines Garprozesses beschrieben.

DE 10 2017 101 183 A1 offenbart ein Verfahren zum Betreiben eines Gargerätes sowie ein Gargerät, bei dem mit einer Heizeinrichtung Gargut in einem Garraum erwärmt wird. Das Gargut wird mit einer Kameraeinrichtung erfasst. Anhand der Erfassung des Garguts wird wenigstens eine Gargutkenngröße ermittelt. Dabei umfasst die Heizeinrichtung eine Heizquelle mit einer Mehrzahl separat ansteuerbarer Heizmittel. Mit jeweils wenigstens einem Heizmittel wird ein räumliches Segment von einer Mehrzahl von räumlichen Segmenten im Garraum gezielt beheizt. Die Ansteuerung der einzelnen Heizmittel wird in Abhängigkeit der Gargutkenngröße vorgenommen.

EP 2 618 634 A1 offenbart ein Mikrowellengerät, bei dem eine örtliche Verteilung mindestens einer Öberflächenbeschaffenheit des Garguts in Form einer Oberflächentemperaturverteilung mittels eines Sensors gemessen wird.

US 2010/0187224 A1 beschreibt mindestens einen Sensor zum Bestimmen einer Oberflächenbeschaffenheit des Garguts in Form einer Temperaturverteilung.

Es ist die **Aufgabe** der vorliegenden Erfindung, die Nachteile des Standes der Technik zumindest teilweise zu überwinden und insbesondere eine konstruktive besonders einfache Möglichkeit bereitzustellen, in dem Garraum befindliches Gargut mit einer gewünschten Verteilung seiner Oberflächeneigenschaft, insbesondere mit einer gleichmäßigen Oberflächeneigenschaft, zu erzeugen.

Diese Aufgabe wird gemäß den Merkmalen der unabhängigen Ansprüche gelöst. Vorteilhafte Ausführungsformen sind Gegenstand der abhängigen Ansprüche, der Beschreibung und der Zeichnungen.

Die Aufgabe wird gelöst durch ein Verfahren zum Behandeln von Gargut in einem Haushalts-Gargerät mit einem Garraum, mindestens einer Gargutbehandlungseinheit und mindestens einem Sensor zum Bestimmen mindestens einer Oberflächenbeschaffenheit des Garguts, bei dem
a) eine örtliche Verteilung mindestens einer Oberflächenbeschaffenheit des Garguts mittels mindestens eines Sensors gemessen wird,
b) aus der gemessenen, dimensionslosen Verteilung der mindestens einen Oberflächenbeschaffenheit und mehreren vorbekannten dimensionslosen örtlichen Leistungsverteilungen im Bereich des Garguts ein jeweiliges Abweichungsmaß berechnet wird, wobei die vorbekannten Leistungsverteilungen unterschiedlichen Sätzen von Einstellwerten der mindestens einen Gargutbehandlungseinheit entsprechen, und
c) die mindestens eine Gargutbehandlungseinheit mit dem Satz von Einstellwerten derjenigen Leistungsverteilung betrieben wird, deren Abweichungsmaß der größten Abweichung zwischen der Verteilungen entspricht.

Dadurch wird der Vorteil erreicht, dass die mindestens eine Oberflächeneigenschaft des Garguts sich besonders präzise und mit nur geringem oder sogar praktisch ohne konstruktiven Mehraufwand einstellen lässt. Speziell kann so Gargut dergestalt behandelt werden, dass es eine hochgradig gleichmäßige Verteilung der Oberflächeneigenschaft erhält. Das Betreiben der mindestens einen Gargutbehandlungseinheit gemäß dem Satz derjenigen Leistungsverteilung von Einstellwerten, deren Abweichungsmaß mindestens ein vorgegebenes Kriterium erfüllt, baut gedanklich auf einer Prognose der voraussichtlichen Entwicklung der Oberflächenbeschaffenheit unter dem neuen Satz von Einstellwerten auf.

Die gemessene Verteilung der mindestens einen Oberflächenbeschaffenheit kann im Folgenden auch als "Messverteilung" bezeichnet werden.

Schritt b) kann auch so verstanden werden, dass die Messverteilung mit mehreren vorbekannten örtlichen Leistungsverteilungen im Bereich des Garguts verglichen wird und als Ergebnis des Vergleichs ein jeweiliges Abweichungsmaß berechnet wird. Das Abweichungsmaß ergibt sich also aus einer Verknüpfung von Werten der Messverteilung mit einer jeweiligen vorbekannten örtlichen Leistungsverteilung.

Es ist eine Ausgestaltung, dass die mindestens eine Oberflächenbeschaffenheit einen Bräunungsgrad oder eine andere Farbänderung des Garguts, eine Oberflächentemperatur und/oder eine Oberflächenfeuchtigkeit des Garguts umfasst. Die Nutzung der anderen Farbänderung als Oberflächenbeschaffenheit kann z.B. für Gargüter sinnvoll sein, die typischerweise keine Bräunung erfahren oder erfahren sollen, z.B. Gemüse, Erbsen oder Brokkoli.

Es ist eine Weiterbildung, dass das Haushalts-Gargerät ein Backofen ist. Der Backofen kann in einer Weiterbildung eine zusätzliche Mikrowellenfunktion und/oder Dampfbehandlungsfunktion aufweisen. Das Haushalts-Gargerät kann alternativ ein Mikrowellengerät sein, das in einer Weiterbildung mindestens einen IR-Strahlungsheizkörper und/oder eine Dampfbehandlungsfunktion aufweist.

Die Gargutbehandlungseinheit ist insbesondere eine Einheit oder Komponente des Haushalts-Gargeräts, die dazu eingerichtet ist, Gargut durch physikalische Einwirkung zu behandeln. Die Gargutbehandlungseinheit kann insbesondere mittels einer Steuereinrichtung des Haushalts-Gargeräts angesteuert werden. Jede Gargutbehandlungseinheit weist mindestens einen Einstellparameter mit jeweils mindestens zwei Einstellwerten auf. Sind nur zwei Einstellwerte einstellbar, kann es sich dabei z.B. um die Einstellungen Aus (z.B. repräsentiert durch den Wert "0") und Ein (z.B. repräsentiert durch den Wert "1") handeln.

Es ist eine Weiterbildung, dass mindestens eine Gargutbehandlungseinheit eine Energieeinbringungseinheit ist, d.h., dass sie dazu eingerichtet ist, Energie zur Behandlung des Garguts in den Garraum einzubringen, z.B. Wärmestrahlung oder Mikrowellen. Dabei ist typischerweise die zugehörige Energieverteilung oder Leistungsverteilung im Garraum räumlich oder örtlich inhomogen. Im Folgenden kann der Ausdruck Leistungsverteilung synonym dem Ausdruck Energieverteilung verwendet werden.

Es ist eine Weiterbildung, dass mindestens eine Gargutbehandlungseinheit eine Stoffeinbringungseinheit ist, die dazu eingerichtet ist, Stoff gerichtet zur lokalen Behandlung des Garguts in den Garraum einzubringen, z.B., Heißluft, Kühlluft und/oder Wasser (z.B. in Form von Wasserdampf oder als Sprühnebel). Auch dabei ist die zugehörige Energieverteilung oder Leistungsverteilung im Garraum typischerweise räumlich oder örtlich inhomogen.

Die vorbekannten örtlichen Leistungsverteilungen können z.B. durch experimentelle Messungen und/oder Simulationsrechnungen, beispielsweise des Herstellers, vorab bereitgestellt werden. Sie werden insbesondere nicht erst während des Ablaufs des Verfahrens erzeugt oder berechnet, sondern sind vorab in einem Datenspeicher (z.B. des Haushaltsgeräts, eines Netzwerkrechners oder in der sog. "Cloud") gespeichert worden.

Die von unterschiedlichen Gargutbehandlungseinheiten erzeugten Leistungsverteilungen im Garraum sind in der Regel unterschiedlich. Die Leistungsverteilung kann zusätzlich oder alternativ auch für eine bestimmte aktivierte Gargutbehandlungseinheit abhängig von den zu ihrem Betrieb gewählten Einstellwerten unterschiedlich sein.

Zur Durchführung des Verfahrens existieren insbesondere mindestens zwei Sätze von Einstellwerten der mindestens einen Gargutbehandlungseinheit, mittels derer sich in dem Garraum unterschiedliche Leistungsverteilungen erzeugen oder einstellen lassen.

Unter einem Satz von Einstellwerten kann insbesondere eine bestimmte Auswahl von Werten der Einstellparameter der mindestens einen Gargutbehandlungseinheit verstanden werden. Der Satz von Einstellwerten kann auch als Wertekonfiguration oder "Maske" bezeichnet werden.

Das Abweichungsmaß kann ein einzelner Wert oder eine Wertemenge sein. Das Abweichungsmaß kann auch als Ähnlichkeitsmaß bezeichnet werden. Insbesondere ist das Abweichungsmaß so beschaffen, dass ein größerer Wert des Abweichungsmaßes einer größeren Abweichung oder eines größeren Unterschieds der Verteilungen entspricht. Insbesondere kann ein Abweichungsmaß des Werts "Null" einer gleichen Verteilung entsprechen, wobei sich dies insbesondere nur auf eine Form der Verteilung (z.B. ausgedrückt in Prozenten eines Maximalwerts) bezieht, nicht auf absolute Werte der Verteilungen.

Erfindungsgemäß wird das Abweichungsmaß aus dimensionslosen (z.B. prozentualen) Messverteilungen (z.B. eines Bräunungsgrads) berechnet. Dies erlaubt einen besonders einfachen Vergleich auch von Verteilungen, die unterschiedliche physikalische Größen (Oberflächenbeschaffenheit, Energie bzw. Leistung) und/oder unterschiedliche Maximalstärken aufweisen.

Das mindestens eine vorgegebene Kriterium kann genau ein Kriterium oder mehrere Kriterien (dann ggf. in vorgegebener Reihenfolge oder Priorisierung) umfassen. Auch können mehrere unterschiedliche Kriterien für unterschiedliche Bereiche des Garguts, für unterschiedliche Gargüter und/oder unterschiedliche Raumbereiche vorliegen.

Es ist eine Weiterbildung, dass das Abweichungsmaß ein vorgegebenes Kriterium erfüllt, wenn es einen vorgegebenen Zielwert erreicht oder (von oben oder von unten kommend) überschreitet.

Erfindungsgemäß entspricht das mindestens eine vorgegebene Kriterium der größten Abweichung zwischen den verglichenen Verteilungen. Dadurch wird eine besonders gleichmäßige Verteilung der Oberflächenbeschaffenheit (z.B. ein gleichmäßiger Bräunungsgrad) auf eine besonders effektive Weise ermöglicht. Bei dieser Ausgestaltung wird also diejenige Leistungsverteilung bestimmt und eingestellt, die sich aus der Gruppe verfügbarerer vorgegebener Leistungsverteilungen am meisten von der Messverteilung unterscheidet bzw. davon abweicht. Dabei wird ausgenutzt, dass in diesem Fall diejenigen Bereiche das Garguts, die geringe Werte der Oberflächenbeschaffenheit wie z.B. eine geringere Bräunung aufweisen (weil sie mit weniger Energie behandelt worden sind als Bereiche des Garguts mit hohen Werten der Oberflächenbeschaffenheit), folgend stärker behandelt (z.B. erwärmt) werden als Bereiche des Garguts mit hohen Werten der Oberflächenbeschaffenheit.

Es ist eine Ausgestaltung, dass das Abweichungsmaß eine Vergleichssumme oder eine Differenzsumme zwischen der Messverteilung und einer Leistungsverteilung ist. So lässt sich das Abweichungsmaß besonders einfach bestimmen. Zudem stellt das Abweichungsmaß dann eine besonders zuverlässige Möglichkeit zur Einschätzung einer Abweichung zwischen zwei Verteilungen dar.

Es ist eine Weiterbildung, dass sich die Vergleichssumme aus einer Multiplikation örtlich entsprechender Werte der Messverteilung und einer Leistungsverteilung und anschließender Addition der zuvor multiplizierten Werte ergibt. Das Abweichungsmaß ist dann für diejenige Verteilung am größten, welche wertemäßig die geringste Vergleichssumme aufweist.

Es ist eine Weiterbildung, dass sich die Differenzsumme aus den Beträgen einer Differenz örtlich entsprechender Werte der Messverteilung und einer Leistungsverteilung und anschließender Addition der zuvor bestimmten Differenzwerte ergibt. Das Abweichungsmaß ist dann für diejenige Verteilung am größten, welche wertemäßig die größte Differenzsumme aufweisen.

Jedoch kann das Abweichungsmaß auch aus anderen Verknüpfungen der Verteilungen, insbesondere aus entsprechenden Werte der Messverteilung und einer Leistungsverteilung, berechnet werden, z.B. nach der Methode der kleinsten Quadrate.

Es ist eine Weiterbildung, dass die Messpunkte der Messverteilung oder der Leistungsverteilung zusätzlich gewichtet werden. Dies kann auch so ausgedrückt werden, dass die Messverteilung oder die Leistungsverteilung mit einer Wichtungsverteilung verknüpft wird. Dies kann so umgesetzt sein, dass die Wichtungsverteilung Wichtungsfaktoren als Werte aufweist, die jeweils mit den entsprechenden lokalen Verteilungswerten multipliziert werden, bevor das Abweichungsmaß bestimmt wird. Die Werte der Wichtungsverteilung können insbesondere in einem Bereich [0; 1] liegen. Diese Weiterbildung ergibt den Vorteil, dass lokale Eigenschaften des Garguts wie eine typische Bräunungsgeschwindigkeit berücksichtigt werden können.

Es ist eine Ausgestaltung, dass die mindestens eine Gargutbehandlungseinheit mindestens eine Gargutbehandlungseinheit aus der Gruppe aufweisend
- mindestens einen elektrischen Strahlungsheizkörper,
- mindestens eine Induktionsspule,
- mindestens eine Mikrowelleneinrichtung,
- mindestens ein strahlgerichtetes Kühlluftgebläse,
- mindestens eine strahlgerichtete Heißlufteinrichtung und/oder
- mindestens eine strahlgerichtete Wassereinspeisungseinrichtung
umfasst. So wird der Vorteil erreicht, dass sich die Oberflächeneigenschaft mit vielen Vorrichtungen (falls in dem Haushalts-Gargerät vorhanden) einzeln oder in beliebiger Kombination vereinheitlichen oder auf eine andere Ziel-Verteilung der Oberflächeneigenschaft einstellen lässt. Dies wiederum erhöht eine Effektivität des Verfahrens. Unter einer strahlgerichteten Vorrichtung kann insbesondere eine Stoffeinbringungseinheit verstanden werden, die dazu eingerichtet ist, mindestens einen lokal begrenzten, gerichteten Strom von Stoff zur lokalen Behandlung des Garguts in den Garraum einzubringen.

Der mindestens eine elektrische Strahlungsheizkörper dient zur Erwärmung des Garraums bzw. des in dem Garraum vorhandenen Garguts. Er kann ein jeweiliger Rohrheizkörper sein, alternativ oder zusätzlich z.B. eine gedruckte Leiterbahn, ein Widerstands-Flächenheizelement usw. Ist das Haushalts-Gargerät mit mindestens einem elektrischen Strahlungsheizkörper ausgestattet, kann das Haushalts-Gargerät in einer Weiterbildung als Backofen und der Garraum als Ofenraum bezeichnet werden.

Der mindestens eine Strahlungsheizkörper kann beispielsweise mindestens einen Unterhitze-Heizkörper zur Erzeugung einer Unterhitze oder Unterhitzefunktion, mindestens einen Oberhitze-Heizkörper zur Erzeugung einer Oberhitze oder Oberhitzefunktion, mindestens einen Grillheizkörper zur Erzeugung einer Grillfunktion (ggf. zusammen mit dem mindestens einen Oberhitze-Heizkörper), einen Ringheizkörper zur Erzeugung einer Heißluft oder Heißluftfunktion, usw. umfassen. Der Einstellparameter eines Strahlungsheizkörpers kann insbesondere unterschiedliche elektrische Leistungen oder Leistungsstufen umfassen, z.B. < 0 W, 200 W, ...., 800 W >.

Es ist eine Ausgestaltung, dass der mindestens eine elektrische Strahlungsheizkörper mindestens zwei Strahlungsheizkörper umfasst und der Satz von Einstellwerten Einstellwerte für mindestens zwei der Strahlungsheizkörper umfasst. In anderen Worten können zur Durchführung des Verfahrens unterschiedliche Leistungsverteilung, die unterschiedlichen Sätzen von Einstellparametern von mindestens zwei Strahlungsheizkörpern entsprechen, genutzt werden.

Es ist eine Weiterbildung, dass die Strahlungsheizkörper einzeln oder individuell betrieben werden können, und zwar insbesondere unabhängig davon, ob mehrere Strahlungsheizkörper bei Auswahl einer bestimmten Betriebsart (z.B. Grillbetriebsart) zusammen betrieben werden. Dies ergibt den Vorteil, dass besonders gut auf das Erreichen einer gewünschten Verteilung der Oberflächeneigenschaft abgestimmte Leistungsverteilungen bereitstellbar sind.

Es ist eine Weiterbildung, dass die Strahlungsheizkörper (insbesondere nur) als funktionale "Betriebsart"-Gruppen oder Heizarten aktivierbar sind, die bestimmten Betriebsarten zugeordnet sind. Dabei kann in einer Variante bei mindestens einer Betriebsart genau ein Strahlungsheizkörper aktivierbar sein bzw. dieser Betriebsart genau ein Strahlungsheizkörper zugeordnet sein. In mindestens einer anderen Betriebsart werden mindestens zwei Strahlungsheizkörper aktiviert bzw. sind dieser anderen Betriebsart mindestens zwei Strahlungsheizkörper zugeordnet. Die zum Vergleich in Schritt b) vorgegebenen örtlichen Leistungsverteilungen können sich dann aus den Leistungseinträgen von zu verschiedenen Betriebsarten zugehörigen Strahlungsheizkörpern ergeben.

Die Mikrowelleneinrichtung kann beispielsweise einen Mikrowellengenerator, eine Mikrowellenführung zur Führung von mittels des Mikrowellengenerators erzeugten Mikrowellen zum Garraum, ggf. mindestens eine - insbesondere drehbare - Antenne und ggf. mindestens einen Wobbler umfassen. Der mindestens eine Einstellparameter der Mikrowelleneinrichtung kann beispielsweise einen oder mehrere der folgenden Einstellparameter umfassen: Mikrowellenleistung, Leistungsverteilung, Mikrowellenfrequenz, Modulation der Mikrowellenfrequenz, Drehwinkel der mindestens einen Antenne, Höhenposition der mindestens einen Antenne, Drehwinkel des mindestens einen Wobblers, Höhenposition des mindestens einen Wobblers. So ändert sich das sog. Modenbild und damit die Leistungsverteilung in dem Garraum mit veränderter Winkelstellung der Drehantenne. Der Mikrowellengenerator ist vorteilhafterweise ein invertergesteuerter Mikrowellengenerator und/oder ein halbleiterbasierter Mikrowellengenerator (Solid State Generator).

Ein möglicher Einstellparameter des strahlgerichteten Kühlluftgebläses kann dessen Drehzahl umfassen. So kann eine ortabhängige Kühlung bestimmter Bereiche des Garguts eingestellt werden. Insbesondere kann das Kühlluftgebläse zur Einbringung eines lokal begrenzten, gerichteten ("strahlgerichteten") Kühlluftstroms in den Garraum eingerichtet sein. Dem Kühlluftgebläse kann dazu mindestens eine Düse oder Klappe in einer Garraumwand zugeordnet sein.

Die strahlgerichtete Heißlufteinrichtung kann einen Strom von Heißluft gezielt lokal auf das Gargut richten. Sie kann beispielsweise als sog. "Air Impingement"-Einrichtung ausgebildet sein, die eine oder mehrerer Düsen zur Einbringung eines lokal begrenzten, gerichteten Heißluftstroms in den Garraum aufweist. Solche Düsen können z.B. einen Durchmesser von 5 mm aufweisen.

Unter einer Wassereinspeisungseinrichtung kann beispielsweise ein Dampferzeuger oder eine Sprühvorrichtung zur gerichteten Einbringung von Sprühnebel, Wasserdampf oder Heißdampf ("Superheated Steam") in den Garraum, insbesondere direkt auf das Gargut, verstanden werden. Mögliche Einstellparameter können z.B. eine Bedampfungs- und/oder Sprühdauer, eine Zielort für den Wasserdampf, Heißdampf und/oder Sprühnebel, eine Anwendungsdauer usw. umfassen. Insbesondere durch die Besprühung bestimmter Bereiche des Garguts kann deren ortabhängige Kühlung erreicht werden.

Es ist eine Ausgestaltung, dass zumindest eine vorbekannte Leistungsverteilung nur für einen Satz von Einstellwerten der mindestens zwei Strahlungsheizkörper bestimmt wird, und z.B. keinen Betrieb der Mikrowelleneinrichtung berücksichtigen, auch falls eine solche Mikrowelleneinrichtung vorhanden sein sollte. Dadurch wird der Vorteil erreicht, dass das Verfahren auch mit einfachen Backöfen ohne weitere Zusatzfunktionen wie einer Mikrowellenfunktion usw. oder für eine Zubereitung von Rezepten, die häufig keinen Mikrowellenbetrieb beschreiben, durchführbar ist.

Jedoch können vorbekannte Leistungsverteilungen zur Durchführung des Verfahrens auch nur unter Berücksichtigung der Einstellparameter der Induktionsspulen, des Mikrowellengenerators usw. bereitgestellt werden.

Es ist eine Ausgestaltung, dass die Schritte a) bis c) zyklisch durchlaufen werden. Dadurch kann eine Überprüfung der aktuellen Oberflächenbeschaffenheit und ggf. deren Korrektur auf eine gewünschte Oberflächenbeschaffenheit durch Änderung der Verteilung der eingebrachten Energie oder Leistung während eines Garablaufs mehrfach vorgenommen, um die gewünschte Oberflächenbeschaffenheit besonders effektiv zu erreichen.

Eine vorgegebene Zeitdauer Δt bei zyklischer Wiederholung des Verfahrens zwischen einem Schritt c) und der folgenden erneuten Durchführung des Schritts a) ist grundsätzlich beliebig wählbar. Sie ist aber zweckmäßigerweise so bemessen, dass sich in Schritt a) Auswirkungen des Betriebs der Energieeinbringungselemente gemäß dem in dem vorhergehenden Schritt c) eingestellten Satz von Einstellwerten feststellen lassen. Die Zeitdauer Δt kann im Bereich von Sekunden oder Minuten liegen, z.B. eine Sekunde, zehn Sekunden, 30 Sekunden, eine Minute, zwei Minuten usw. betragen. Die Zeitdauer Δt kann fest oder variabel sein. Zur vorteilhaften Vermeidung unnötiger Einstellungen neuer Sätze von Einstellparametern zu Beginn eines Garvorgangs, wenn das Garguts noch kaum gebräunt ist, aber zur Berücksichtigung schneller Bräunungsänderungen bei fortgeschrittener Behandlungsdauer ist es vorteilhaft, wenn die Zeitdauer Δt mit fortschreitender Behandlungsdauer verkürzt wird. Die Zeitdauer Δt kann auch als Zyklendauer bezeichnet werden.

Ein das Verfahren nutzender Garablauf oder Garabschnitt kann mit Eintritt mindestens eines Ereignisses beendet werden, z.B. mit Erreichen einer vorgegebenen Gardauer, mit nutzerseitiger Beendigung des Garablaufs, mit Erreichen einer Ziel-Oberflächenbeschaffenheit wie einem maximalen oder durchschnittlichen Ziel-Bräunungsgrad, usw.

In einer Weiterbildung wird zu Beginn eines Garablaufs eine bestimmter erster Satz von Einstellwerten vorgegeben und die Energieeinbringungselemente zunächst gemäß diesem Anfangssatz von Einstellwerten betrieben. Der Anfangssatz kann fest vorgegeben sein oder von der Art des Garguts, der Ziel-Garraumtemperatur usw. abhängig sein.

Es ist eine Ausgestaltung, dass der mindestens eine Sensor mindestens einen optischen Sensor und/oder mindestens einen Infrarotsensor umfasst. So lässt sich eine Oberflächenbeschaffenheit besonders zuverlässig bestimmen und effektiv auswerten. Der optische Sensor eignet sich besonders zur Bestimmung eines Bräunungsgrads und/oder einer Bestimmung der Feuchtigkeit auf der Oberfläche des Garguts, während der Infrarotsensor sich besonders zur Bestimmung einer Temperaturverteilung auf der Oberfläche des Garguts eignet. Der Infrarotsensor ist insbesondere in einem nahen Infrarotbereich (NIR) empfindlich.

Es ist eine Weiterbildung, dass aus den Messwerten des mindestens einen Sensors eine ortsaufgelöste, insbesondere bildpunktartige, Verteilung der Oberflächenbeschaffenheit bereitgestellt wird, insbesondere als zweidimensionales Bild. Dazu kann mindestens ein Sensor ein ortsauflösend messender Sensors sein. Dies ermöglicht vorteilhafterweise eine besonders schnelle Durchführung des Verfahrens. Alternativ oder zusätzlich kann mindestens ein Sensor relativ zu dem Gargut bewegt werden (z.B. durch Befestigung auf einem verfahrbaren Träger) und an unterschiedlichen Raumpositionen Messungen durchführen, welche zu einem Gesamt-Bild zusammengeführt werden. So wird der Vorteil erreicht, dass die Oberfläche insbesondere auch von voluminösem oder nicht flachem Gargut vollständiger erfassbar oder ausmessbar ist. Alternativ oder zusätzlich können auch mehrere aus unterschiedlichen Blickwinkeln und/oder an unterschiedlichen Positionen in den Garraum gerichtete Sensoren verwendet werden, deren Messungen z.B. zu einem Gesamt-Bild zusammengeführt werden können.

Die vorgegebenen Leistungsverteilungen können ebenfalls als zweidimensionale, insbesondere horizontal ausgerichtete Ebenen definiert sein oder vorliegen. Allgemein können die Leistungsverteilungen für einen bestimmten Satz von Einstellwerten als zu unterschiedlichen Höhenpositionen im Garraum zugeordnete Ebenen ("Ebenenschar") zugehörig vorliegen. Für eine bestimmte Höhenposition oder Ebene (oder allgemein für eine beliebige Raumausrichtung) können dann zweidimensionale Darstellungen unterschiedlicher Sätze von Einstellwerten für eine bestimmte Höhenposition vorliegen. Die Höhenposition entspricht vorteilhafterweise zumindest ungefähr der Höhenposition des Garguts.

Zusätzlich oder alternativ kann auch das Gargut bewegt werden, um seine Oberflächeneigenschaft(en) zu messen. So kann das Gargut auf einen Drehteller gelegt werden. Zusätzlich oder alternativ kann das Gargut in dem Garraum höhenverstellbar sein, z.B. durch eine - insbesondere motorisch - höhenverstellbare Halterung für einen Gargutträger oder durch einen höhenverstellbaren Gargutträger. Die Höhenverstellung des Garguts erfolgt insbesondere automatisch durch das Haushaltsgargerät.

Es ist eine Weiterbildung, dass der mindestens eine optische Sensor eine Digitalkamera umfasst oder ist. Diese ist vorteilhafterweise eine Farbkamera, kann aber auch eine Schwarz-Weiß-Kamera sein.

Es ist eine Weiterbildung, dass der mindestens ein Infrarotsensor mindestens eine IR-Kamera (auch als Wärmebildkamera bezeichnet) umfasst oder ist. Er kann aber zusätzlich oder alternativ als mindestens eine sog. Thermosäule oder "Thermopile" usw. ausgebildet sein. Der mindestens ein Infrarotsensor kann aus als IR-Spektroskop ausgebildet sein.

Es ist eine Weiterbildung, dass die mindestens eine Gargutbehandlungseinheit mindestens zwei Strahlungsheizkörper (insbesondere Rohrheizkörper) und mindestens eine optische Digitalkamera zum Bestimmen einer Verteilung des Bräunungsgrads des Garguts aufweist, wobei
a) ein bildpunktartig aufgebautes Bild des Garguts aufgenommen wird, das die örtliche Verteilung des Bräunungsgrads des Garguts zeigt. Die Verteilung des Bräunungsgrads kann z.B. als zweidimensionales Bildpunktfeld mit den Helligkeiten als Bildpunktwerten vorliegen,
b) aus der gemessenen Verteilung des Bräunungsgrads und mehreren vorbekannten örtlichen Leistungsverteilungen der Strahlungsheizkörper im Bereich des Garguts ein jeweiliges Abweichungsmaß berechnet, wobei die vorbekannten Leistungsverteilungen unterschiedlichen Sätzen von Einstellwerten der Strahlungsheizkörper entsprechen. Auch die Leistungsverteilungen der Strahlungsheizkörper liegen vorteilhafterweise als zweidimensionales Wertefeld vor, wobei die einzelnen Werte den örtlichen Leistungen entsprechen. Vorteilhafterweise weisen die zweidimensionalen Wertefelder die gleiche Zahl und Anordnung von Wertepunkten auf wie das zweidimensionale Bildpunktfeld, und
c) die Strahlungsheizkörper mit dem Satz von Einstellwerten derjenigen Leistungsverteilung betrieben werden, deren Abweichungsmaß maximal ist, sich also maximal von der Verteilung des Bräunungsgrads unterscheidet.

Die obige Weiterbildung kann zusätzlich oder alternativ unter Verwendung mindestens einer Wärmebildkamera zum Bestimmen einer Oberflächentemperaturverteilung des Garguts ablaufen, wobei dann die Verteilung der Oberflächentemperatur z.B. als zweidimensionales Bildpunktfeld mit den lokalen Temperaturen als Bildpunktwerten vorliegen kann.

Allgemein kann bei bildpunktartigen Verteilungen die Vergleichssumme aus einer Multiplikation der Werte sich örtlich entsprechender Bildpunkte der beiden zu verknüpfenden Verteilungen (bildpunktweise Multiplikation) und anschließender Addition der zuvor multiplizierten Werte berechnet werden. Der Differenzwert kann aus dem Betrag der Differenz örtlich entsprechender Bildpunktwerte der zu verknüpfenden Verteilungen und anschließender Addition der Differenzwerte berechnet werden.

Jedoch kann das Abweichungsmaß auch aus anderen Verknüpfungen entsprechender Werte der beiden zu verknüpfenden Verteilungen berechnet werden, z.B. nach der Methode der kleinsten Quadrate, u.v.m.

Es ist eine Ausgestaltung, dass eine Position des Garguts bzw. seiner Oberfläche in dem Garraum bestimmt wird und nur die sich auf diese Position beziehenden örtlichen Leistungsverteilungen und Messverteilungen bzw. Teilbereiche davon zur Durchführung des Verfahrens verwendet werden. So ergibt sich der Vorteil, dass Messwerte der Oberfläche des Garguts von Messwerten anderer Oberflächen separiert werden können und dadurch der Einfluss der anderen Oberflächen auf das Verfahren erheblich reduziert und ggf. sogar ganz ausgeschlossen werden kann. Die Bildauswertung kann anhand eines optischen Bild und/oder eines Wärmebilds durchgeführt werden.

Im Folgenden können unter "Position" insbesondere mindestens ein Positionswert und/oder eine Ausdehnung des Garguts in dem Garraum verstanden werden. Die Ausdehnung kann eine räumliche Ausdehnung und/oder eine Ausdehnung in mindestens einer bestimmten Raumrichtung (z.B. in Höhenrichtung und/oder lateraler Richtung) verstanden werden. Die Ausdehnung kann z.B. einer konturartigen Ausdehnung ("Außenkontur") oder einer maximalen Ausdehnung entsprechen. Durch diese Ausgestaltung wird der Vorteil erreicht, dass die örtlichen Leistungsverteilungen besonders genau an die Oberfläche des Garguts angepasst werden können. Insbesondere wird dabei berücksichtigt, dass die Leistungsverteilung abhängig von der Position des Garguts im Garraum unterschiedlich ausfallen kann. In dem Verfahren wird also die Messverteilung mit den Leistungsverteilungen nur im Bereich des Garguts bzw. der Position des Garguts verglichen. So können z.B. nur Bildbereiche, die das Gargut enthalten, ausgewertet und miteinander verglichen werden.

Es ist eine Weiterbildung, dass die Position, insbesondere auch Ausdehnung, des Garguts bzw. seiner Oberfläche durch eine Bildauswertung bestimmt wird. So kann die Oberfläche des Garguts von anderen Flächen in dem Garraum wie einem Gargutträger und/oder Wänden einer Wandung des Garraums (auch als Muffel bezeichnet) unterschieden werden. Insbesondere ergibt sich der Vorteil, dass Messwerte der Oberfläche des Garguts von Messwerten anderer Oberflächen besonders einfach separiert werden können. Die Bildauswertung kann anhand eines optischen Bild und/oder eines Wärmebilds durchgeführt werden.

Es ist eine Weiterbildung, dass die Bildauswertung eine Objekterkennung des Garguts umfasst. Dies ermöglicht eine besonders präzise, automatische Bestimmung der Position des Garguts.

Die Position oder Ausdehnung der Oberfläche des Garguts in dem Garraum kann alternativ oder zusätzlich durch eine Auswertung spektraler (optischer und/oder thermischer) Änderungen zu Beginn des Garprozesses bestimmt werden. So wird sich die Oberfläche des Garguts in der Regel langsamer erwärmen als ein typischerweise metallischer Gargutträger, was in einer Bildfolge erkennbar ist und auswertbar ist. Alternativ oder zusätzlich können zeitliche Änderungen in der wellenlängenabhängigen Reflektion ausgewertet werden.

Alternativ kann die Position des Garguts auf andere Weise bestimmt werden, z.B. nutzerseitig. Beispielsweise kann in einer Weiterbildung ein optisches Bild des Garraums aufgenommen werden und einem Nutzer zur Ansicht bereitgestellt werden, z.B. auf einem berührungsempfindlichen Bildschirm, beispielsweise des Haushalts-Gargerät und/oder einem Nutzerendgerät wie einem Smartphone oder Tablet-PC. Der Nutzer kann nun diejenige Bildfläche bestimmen, die dem Gargut entspricht. Dies kann beispielsweise durch Entlangfahren der durch den Nutzer erkannten Kontur des Garguts mittels eines Fingers oder Stifts auf dem berührungsempfindlichen Bildschirm erfolgen. Alternativ kann das aufgenommene Bild bildlich in Segmente unterteilt werden, und ein Nutzer kann diejenigen Segmente auswählen, auf denen das Gargut gezeigt ist, insbesondere auf denen das Gargut überwiegend gezeigt ist, insbesondere auf denen nur das Gargut gezeigt ist. Das Haushalts-Gargerät kann folgend nur die nutzerseitig ausgewählten Segmente zur Durchführung des Verfahrens nutzen.

Es ist noch eine Weiterbildung, dass die Höhenposition durch Bestimmung einer Einschubebene eines das Gargut tragenden Gargutträgers (Gitterrosts, Backblechs usw.) bestimmt wird. Dabei kann als Höhe des Garguts die Höhe der Einschubebene oder die Höhe der Einschubebene zuzüglich eines bestimmten Höhenmaßes ("Zusatzhöhe") bestimmt werden. Ist beispielsweise bekannt, dass das Gargut Pizza ist, kann auf die Höhe der Einschubebene eine Zusatzhöhe von 1 cm hinzugefügt werden, bei einem Auflauf z.B. 3 cm usw.

Es ist eine Ausgestaltung, dass eine Position des Garguts bestimmt wird und die vorgegebenen Leistungsverteilungen an die bestimmte Position des Garguts angepasst werden. Dabei kann die Position des Garguts für das Verfahren einmal zu Anfang bestimmt und dann für die Dauer des Verfahrens, insbesondere für mehrere Zyklen, als gegeben angenommen werden. Alternativ kann die Position, insbesondere Ausdehnung, des Garguts mehrfach während des Verfahrens bestimmt werden, z.B. für jeden Zyklus oder nach jedem n-ten, n > 1, Zyklus. So wird der Vorteil erreicht, dass die Leistungseinstellung besonders präzise an die aktuelle Lage der Oberfläche des Garguts angepasst werden kann, z.B. indem nur diejenigen Leistungsverteilungen bei der Durchführung des Verfahrens berücksichtigt werden, die der bestimmten Position des Garguts zumindest annähernd entsprechen. Dies ist besonders vorteilhaft für Gargut, dessen Form sich während der Behandlung merklich ändert, z.B. Kuchen oder Brot.

Es ist eine Ausgestaltung, dass die Höhenposition des Garguts variierbar ist. Dies ergibt den Vorteil, dass so die Lage des Garguts an besonders vorteilhafte Leistungsverteilungen anpassbar ist. Dabei wird ausgenutzt, dass Gargutbehandlungseinheiten abhängig von ihrer Entfernung zum Gargut eine stärker homogene oder inhomogene Leistungsverteilung in den Garraum einbringen. Meist ist die Leistungsverteilung dabei umso inhomogener je geringer die Entfernung ist. Ist nun beispielsweise bekannt oder wird vermutet, dass sich eine gewünschte - insbesondere größte - Abweichung mit stärker inhomogenen vorgegebenen Leistungsverteilungen effektiver erreichen lässt, ist es vorteilhaft, das Gargut näher an mindestens einer Gargutbehandlungseinheit zu positionieren. Befindet sich die mindestens eine Gargutbehandlungseinheit im Bereich einer Decke des Garraums (was z.B. typisch für eine Anordnung eines Oberhitze-Heizkörpers und eines Grillheizkörpers ist), kann das Gargut z.B. durch Höhenverstellung näher in Richtung der Decke gebracht werden als ursprünglich vorgesehen. Es ist also eine Weiterbildung, dass die Höhenposition des Garguts zur Positionsübereinstimmung mit einer bestimmten, zu einer Ziel-Höhenposition des Garguts passenden Gruppe von vorgegebenen Leistungsverteilungen einstellbar ist.

Die Variation der Höhenposition kann automatisch durch das Haushalts-Gargerät erfolgen (beispielsweise durch Betätigen einer Höhenverstelleinrichtung eines Gargutträgers oder für einen Gargutträger) und/oder kann durch einen Nutzer manuell erfolgen, z.B. nach Ausgabe eines entsprechenden Hinweises an den Nutzer.

Allgemein kann das Verfahren auch durchgeführt werden, wenn sich gleichzeitig mehrere Gargüter oder Speisen in dem Garraum befinden. Das Verfahren kann dann unter Berücksichtigung nur eines Garguts, einer Teilmenge der Gargüter oder für alle Gargüter durchgeführt werden. Für den Fall, dass das Verfahren unter Berücksichtigung mehrerer Gargüter durchgeführt werden soll, kann das Abweichungsmaß so bestimmt werden, dass die jeweils gewünschte mindestens eine (Ziel-)Verteilung der Oberflächeneigenschaft(en) für alle zu berücksichtigenden Gargüter möglichst gut angenähert wird. In einem Beispiel soll für mehrere Gargüter ein jeweiliger möglichst gleichmäßiger Bräunungsgrad an ihrer Oberfläche erreicht werden, wobei in einer Variante unterschiedliche Gargüter unterschiedliche Ziel-Bräunungsgrade aufweisen können. Die Bestimmung des Abweichungsmaßes erfolgt unter gemeinsamer Betrachtung der Bildpunkte aller zu berücksichtigenden Gargüter. Sind unterschiedliche Ziel-Bräunungsgrade für unterschiedliche Gargüter erwünscht, kann das Abweichungsmaß und/oder der zugehörige Satz von Einstellwerten daran angepasst werden. Beispielsweise können Gargüter, die weniger gebräunt werden sollen und/oder die zur Erreichung ihres Ziel-Bräunungsgrads einen vergleichsweise geringen Leistungseintrag benötigen als andere Gargüter, mit einer geringeren Leistung beaufschlagt werden als Bereiche, die stärker gebräunt werden sollen. Dies kann z.B. mittels einer geeigneten Wichtungsverteilung eingestellt werden. Diese Ausgestaltung kann analog an andere Oberflächeneigenschaften (z.B. eine Oberflächentemperatur usw.) und/oder an andere gewünschte (Ziel-) Verteilungen der mindestens einen Oberflächeneigenschaft der mehreren Gargüter angepasst werden.

Diese Berücksichtigung unterschiedlicher Ziel-Bräunungsgrade für unterschiedliche Bereiche von Gargut in dem Garraum während der Durchführung des Verfahrens kann analog auf mindestens ein Gargut angewandt werden, dessen Oberfläche eine nicht-homogene Verteilung seiner Oberflächeneigenschaft(en) erhalten soll, z.B. eine vorgegebene nicht-homogene Bräunungsverteilung . Die Oberfläche des Garguts kann dann (z.B. automatisch oder durch einen Nutzer) in unterschiedliche Teilbereiche oder Segmente eingeteilt werden und dann das Verfahren unter Berücksichtigung unterschiedlicher Ziel-Oberflächeneigenschaften durchgeführt werden. Beispielsweise können Teilbereiche des Garguts, die weniger gebräunt werden sollen und/oder die zur Erreichung ihres Ziel-Bräunungsgrads einen vergleichsweise geringen Leistungseintrag benötigen als andere Teilbereiche, mit einer geringeren Leistung beaufschlagt werden als Teilbereiche, die stärker gebräunt werden sollen. Dies kann z.B. mittels einer geeigneten Wichtungsverteilung eingestellt werden. Diese Ausgestaltung weist den Vorteil auf, dass auch hierfür als Kriterium für das Abweichungsmaß eine größte Abweichung zur Auswahl einer geeigneten Leistungsverteilung verwendet werden kann.

Die Aufgabe wird auch gelöst durch ein Haushalts-Gargerät, das dazu eingerichtet ist, das oben beschriebene Verfahren ablaufen zu lassen. Das Haushalts-Gargerät kann analog zu dem Verfahren ausgebildet sein und weist die gleichen Vorteile auf.

Insbesondere kann das Haushalts-Gargerät einen Garraum, mindestens eine Gargutbehandlungseinheit, mindestens einen Sensor zum Bestimmen einer mindestens einer Oberflächenbeschaffenheit des Garguts, mindestens eine Auswerteeinrichtung und eine Steuereinrichtung zum Ansteuern der mindestens einer Gargutbehandlungseinheit gemäß der Einstellwerte aufweisen.

Es ist eine Ausgestaltung, dass die Auswerteeinrichtung dazu eingerichtet ist, zumindest Schritt b) des Verfahrens durchzuführen. Schritt c) wird insbesondere mittels der Steuereinheit ausgeführt. Auch kann die Steuereinrichtung Schritt a) auslösen.

Allgemein können die Auswerteeinrichtung und die Steuereinrichtung in einer Funktionseinheit integriert sein. So kann die Steuereinrichtung auch als Auswerteeinrichtung dienen oder eingerichtet sein.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden schematischen Beschreibung eines Ausführungsbeispiels, das im Zusammenhang mit den Zeichnungen näher erläutert wird.
- Fig.1: zeigt als Schnittdarstellung in Seitenansicht eine Skizze eines Haushalts-Gargeräts, das zum Ablauf des oben beschriebenen Verfahrens eingerichtet ist;
- Fig.2: zeigt einen Verfahrensablauf zum Durchführen des oben beschriebenen Verfahrens auf dem Haushalts-Gargerät; und
- Fig.3A bis 3E: zeigen verschiedene zu dem Verfahren zugehörige Mess- und Leistungsverteilungen.

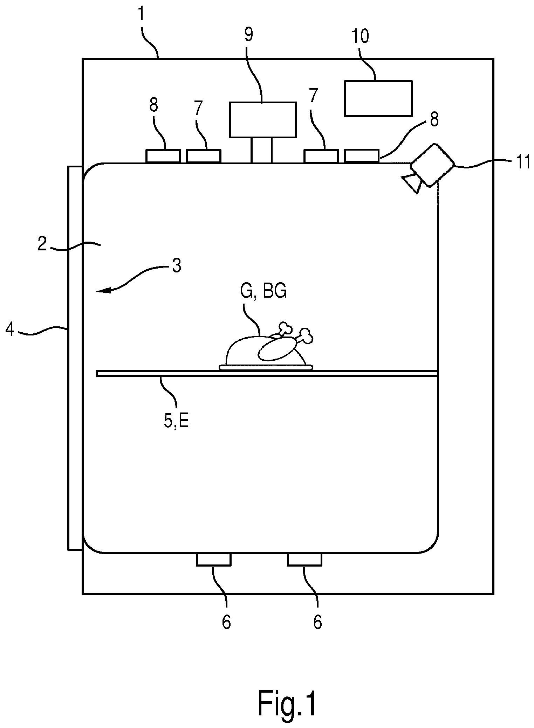

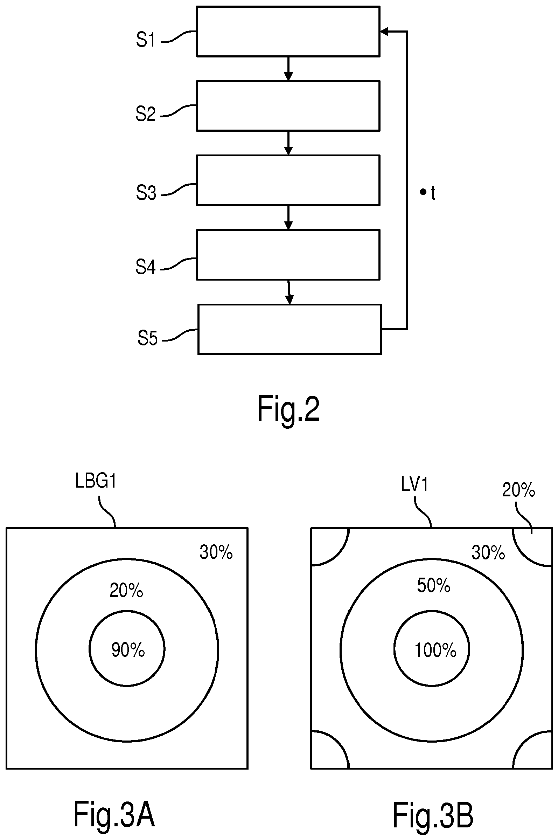

**Fig.1** zeigt als Schnittdarstellung in Seitenansicht eine Skizze eines Haushalts-Gargeräts 1, das zum Ablauf des in Fig.2 näher beschriebenen Verfahrens eingerichtet ist. Das Haushalts-Gargerät 1 weist einen Garraum 2 mit einer vorderseitigen Beschickungsöffnung 3, die mittels einer Tür 4 verschließbar ist, auf. In dem Garraum 2 ist auf einem Gargutträger 5 Gargut G angeordnet. Der Gargutträger 5 ist auf einer von mehreren möglichen Einschubebenen E angeordnet. Das Haushalts-Gargerät 1 kann dazu eingerichtet sein, automatisch festzustellen, welche Einschubebenen E belegt ist.

Das Haushalts-Gargerät 1 weist ferner mehrere Gargutbehandlungseinheiten in Form von IR-Strahlungsheizkörpern 6 bis 8 auf, nämlich einen hier ringförmig eingezeichneten Unterhitze-Heizkörper 6, einen ringförmig eingezeichneten Oberhitze-Heizkörper 7 und einen ringförmig eingezeichneten Grillheizkörper 8.

Zusätzlich oder alternativ kann eine Gargutbehandlungseinheit in Form einer Mikrowellen-Erzeugungseinrichtung 9 vorhanden sein. Die Mikrowellen-Erzeugungseinrichtung 9 kann z.B. einen invertergesteuerten Mikrowellengenerator, eine dreh- und/oder höhenverstellbare Antenne und/oder einen dreh- und/oder höhenverstellbaren Wobbler (o. Abb.) aufweisen.

Die Gargutbehandlungseinheiten 6 bis 9 werden mittels einer Steuereinheit 10 angesteuert. Die Steuereinheit 10 ist zudem mit einem optischen Sensor in Form einer Digitalkamera 11 verbunden. Die Digitalkamera 11 ist so angeordnet, dass sie in den Garraum 2 gerichtet ist und in Bild des Garguts G aufnehmen kann. Dadurch kann die Digitalkamera 11 zum Bestimmen eines Bräunungsgrads BG des Garguts G als der Oberflächenbeschaffenheit verwendet werden.

Die Steuereinheit 10 kann zudem dazu eingerichtet sei, das oben beschriebene Verfahren durchzuführen und kann dazu auch als Auswerteeinrichtung dienen. Alternativ kann die Auswertung auf einer geräteexternen Instanz wie einem Netzwerkrechner oder der sog. "Cloud" ablaufen (o. Abb.).

**Fig.2** zeigt einen Verfahrensablauf zum Durchführen des Verfahrens auf dem Haushalts-Gargerät 1 unter der beispielhaften Annahme, dass das Haushalts-Gargerät 1 keine Mikrowellen-Erzeugungseinrichtung 9 aufweist oder diese nicht nutzt, z.B. weil sie deaktiviert ist. Auch sei angenommen, dass sich nur ein Gargut G in dem Garraum 2 befindet.

In einem Schritt S1 wird eine örtliche Verteilung des Bräunungsgrads BG des Garguts G dadurch gemessen, dass die Digitalkamera 11 ein Bild des Garguts G aufnimmt.

In einem zweiten Schritt S2 wird mittels der Steuereinheit 10 eine Objekterkennung in dem von der Digitalkamera 11 aufgenommenen Bild durchgeführt und dadurch der der Bildbereich identifiziert, der dem Gargut G entspricht bzw. in dem sich das Gargut G befindet.

In einem dritten Schritt S3 wird die für den Bildbereich des Garguts G gemessene Verteilung des Bräunungsgrads für den gleichen Bereich mit vorbekannten örtlichen Leistungsverteilungen (z.B. Wärmeeinträgen) verknüpft, indem für diesen Bildbereich ein Abweichungsmaß in Form z.B. einer Vergleichssumme oder eines Differenzwerts bestimmt wird. Dabei entsprechen die vorbekannten örtlichen Leistungsverteilungen unterschiedlichen Sätzen von Einstellwerten der Strahlungsheizkörper 6 bis 8 im Bereich des Garguts G. Dabei kann die Kenntnis der Einschubebene E und ggf. der Art des Garguts G (z.B. Hähnchen, Pizza usw.) dazu verwendet werden, die vorbekannten örtlichen Leistungsverteilungen für die dadurch zumindest ungefähr bekannte Höhe des Garguts G in dem Garraum 2 zu verwenden.

Die Leistungsverteilungen können z.B. als zweidimensionale, insbesondere horizontal ausgerichtete Schnittebenen durch den Garraum 2 vorliegen. Allgemein können die Leistungsverteilungen für einen bestimmten Satz von Einstellwerten als zu unterschiedlichen Höhen zugeordnete Ebenen ("Ebenenschar") vorliegen.

In einem vierten Schritt S4 wird aus den zuvor für die unterschiedlichen Leistungsverteilungen bestimmten Werten der Abweichungsmaße das Abweichungsmaß mit dem Wert ausgesucht, der eine höchste Unterschiedlichkeit zwischen der Messverteilung und der Leistungsverteilung ausdrückt. Diese Leistungsverteilung ist diejenige Leistungsverteilung, die, wenn sie in den Garraum 2 eingeprägt wird, das Gargut G am effektivsten in Richtung einer einheitlichen Bräunungsverteilung hin behandelt. Der ausgesuchte Wert kann z.B. der kleinste Wert einer Vergleichssumme oder der höchste Wert einer Differenzsumme sein.

In einem Schritt S5 werden die Strahlungsheizkörper 6 bis 8 mit dem Satz von Einstellwerten derjenigen Leistungsverteilung betrieben, die dem ausgesuchten Wert des Abweichungsmaßes entspricht.

Die Schritte S1 bis S5 können mit einer Zyklendauer Δt wiederholt werden.

**Fig.3A** zeigt eine erste Messverteilung LBG1 des Bräunungsgrads BG des Garguts G in Prozent. Dabei kann ein Wert von 100 % z.B. einem maximalen gemessenen Bräunungsgrad BG oder, wie dargestellt, einem Ziel-Bräunungsgrad BG entsprechen.

**Fig.3B** zeigt eine zu der Einschubebene E bzw. zur der Höhe des Garguts G gehörige Leistungsverteilung LV1 am Ort bzw. an der Position des Garguts G für einen Satz von Einstellwerten, bei denen nur die Unterhitze-Heizfunktion und die Oberhitzeheizfunktion aktiviert sind, der Grillheizkörper 8 jedoch ausgeschaltet ist (Einstellwert = 0).

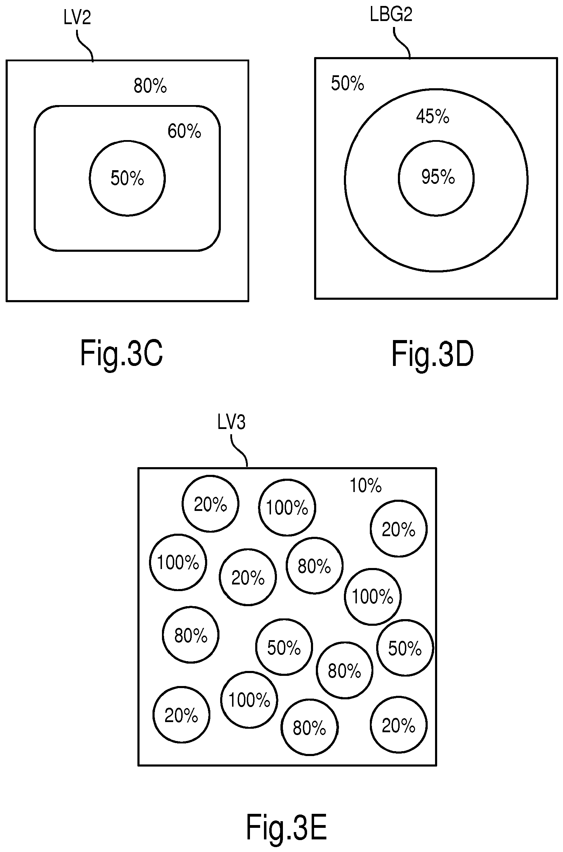

**Fig.3C** zeigt eine zu der Einschubebene E bzw. zur der Höhe des Garguts G gehörige Leistungsverteilung LV2 am Ort bzw. an der Position des Garguts G für einen Satz von Einstellwerten, bei denen nun auch der Grillheizkörper 8 aktiviert ist. Die Form der Leistungsverteilung LV2 unterscheidet sich stärker von der Messverteilung LBG1 des Bräunungsgrads BG als die Leistungsverteilung LV1.

Durch in Fig.2 beschriebene Verfahren würde sich ergeben, dass das Abweichungsmaß der Leistungsverteilung LV2 eine stärkere Abweichung ergibt als das Abweichungsmaß der Leistungsverteilung LV1. Somit würden in Schritt S5 die Strahlungsheizkörper 6 bis 8 mit dem zu der Leistungsverteilung LV2 gehörigen Satz von Einstellwerten betrieben werden.

**Fig.3D** zeigt eine zweite Messverteilung LBG2 des Bräunungsgrads BG des Garguts G in Prozent, nachdem das Gargut G für einen gewissen Zeitraum (z.B. der Zyklenzeit Δt) der Leistungsverteilung LV2 ausgesetzt war. Dadurch ist der Bräunungsgrad BG des Garguts G vereinheitlicht worden, was einem gewünschten Garergebnis einer homogenen Verteilung des Bräunungsgrads BG auf der Oberfläche des Garguts 2 näherkommt als die Verteilung des Bräunungsgrads LBG1 aus Fig.3A.

**Fig.3E** zeigt eine weitere mögliche Leistungsverteilung LV3, die mittels einer Einstrahlung von Mikrowellen durch die Mikrowellen-Erzeugungsvorrichtung 9 in dem Garraum 2 erzeugt worden ist.

Selbstverständlich ist die vorliegende Erfindung nicht auf das gezeigte Ausführungsbeispiel beschränkt.

Allgemein kann unter "ein", "eine" usw. eine Einzahl oder eine Mehrzahl verstanden werden, insbesondere im Sinne von "mindestens ein" oder "ein oder mehrere" usw., solange dies nicht explizit ausgeschlossen ist, z.B. durch den Ausdruck "genau ein" usw.

Auch kann eine Zahlenangabe genau die angegebene Zahl als auch einen üblichen Toleranzbereich umfassen, solange dies nicht explizit ausgeschlossen ist.

### Bezugszeichenliste

- 1: Haushalts-Gargerät
- 2: Garraum
- 3: Beschickungsöffnung
- 4: Tür
- 5: Gargutträger
- 6: Unterhitze-Heizkörper
- 7: Oberhitze-Heizkörper
- 8: Grillheizkörper
- 9: Mikrowellen-Erzeugungseinrichtung
- 10: Steuereinheit
- 11: Digitalkamera
- BG: Bräunungsgrad
- E: Einschubebene
- LBG1: Verteilung des Bräunungsgrads
- LBG2: Verteilung des Bräunungsgrads
- LV1: Leistungsverteilung
- LV2: Leistungsverteilung
- LV3: Leistungsverteilung
- G: Gargut
- S1-S5: Verfahrensschritte
- Δt: Zyklendauer

## Patentansprüche

1. Verfahren (S1-S5) zum Behandeln von Gargut (G) in einem Haushalts-Gargerät (1) mit einem Garraum (2), mindestens einer Gargutbehandlungseinheit (6-9) und mindestens einem Sensor (11) zum Bestimmen mindestens einer Oberflächenbeschaffenheit (BG) des Garguts (G), bei dem
a) eine örtliche Verteilung (LBG1, LBG2) mindestens einer Oberflächenbeschaffenheit (BG) des Garguts (G) mittels mindestens eines Sensors (11) gemessen wird (S1),
b) aus der gemessenen, dimensionslosen Verteilung (LBG1, LBG2) der mindestens einen Oberflächenbeschaffenheit (BG) und mehreren vorbekannten dimensionslosen örtlichen Leistungsverteilungen (LV1, LV2) im Bereich des Garguts (G) ein jeweiliges Abweichungsmaß berechnet wird, wobei die vorbekannten Leistungsverteilungen (LV1, LV2) unterschiedlichen Sätzen von Einstellwerten der mindestens einen Gargutbehandlungseinheit (6-9) entsprechen (S3), und
c) die mindestens eine Gargutbehandlungseinheit (6-9) mit dem Satz von Einstellwerten derjenigen Leistungsverteilung (LV2) betrieben wird, deren Abweichungsmaß der größten Abweichung zwischen den Verteilungen (LBG1, LBG2, LV1, LV2) entspricht.

2. Verfahren (S1-S5) nach einem der vorhergehenden Ansprüche, bei dem das Abweichungsmaß eine Vergleichssumme oder eine Differenzsumme zwischen den Verteilungen ist.

3. Verfahren (S1-S5) nach einem der vorhergehenden Ansprüche, bei dem die mindestens eine Gargutbehandlungseinheit (6-9) mindestens eine Gargutbehandlungseinheit aus der Gruppe aufweisend
- mindestens einen elektrischen Strahlungsheizkörper (6-8),
- mindestens eine Induktionsspule,
- mindestens eine Mikrowelleneinrichtung (9),
- mindestens eine strahlgerichtete Heißlufteinrichtung,
umfasst.

4. Verfahren (S1-S5) nach einem der vorhergehenden Ansprüche, bei dem die mindestens eine Gargutbehandlungseinheit (6-9) zusätzlich mindestens eine Gargutbehandlungseinheit aus der Gruppe aufweisend
- mindestens ein strahlgerichtetes Kühlluftgebläse,
- mindestens eine strahlgerichtete Wassereinspeisungseinrichtung
umfasst.

5. Verfahren (S1-S5) nach einem der Ansprüche 3 bis 4, bei dem der mindestens eine elektrische Strahlungsheizkörper (6-8) mindestens zwei Strahlungsheizkörper (6-8), insbesondere aus der Gruppe Unterhitzeheizkörper (6), Oberhitzeheizkörper (7) und Grillheizkörper (8), umfasst und der Satz von Einstellwerten Einstellwerte für mindestens zwei der Strahlungsheizkörper (6-8) umfasst.

6. Verfahren (S1-S5) nach Anspruch 5, bei dem die vorbekannte Leistungsverteilung (LV1, LV2) nur für einen Satz von Einstellwerten der mindestens zwei Strahlungsheizkörper (6-8) bestimmt wird.

7. Verfahren (S1-S5) nach einem der vorhergehenden Ansprüche, bei dem die Schritte a) bis c) zyklisch durchlaufen werden.

8. Verfahren (S1-S5) nach einem der vorhergehenden Ansprüche, bei dem der mindestens eine Sensor (11) mindestens einen optischer Sensor und/oder mindestens einen Infrarotsensor umfasst.

9. Verfahren (S1-S5) nach einem der vorhergehenden Ansprüche, bei dem die mindestens eine Oberflächenbeschaffenheit einen Bräunungsgrad, eine Oberflächentemperatur und/oder eine Feuchtigkeit umfasst.

10. Verfahren (S1-S5) nach einem der vorhergehenden Ansprüche, bei dem eine Position (E) des Garguts (G) in dem Garraum (2) bestimmt wird und nur die sich auf diese Position beziehenden örtlichen Leistungsverteilungen (LV1, LV2) und Verteilungen (LBG1, LBG2) der Oberflächenbeschaffenheit (BG) zur Durchführung des Verfahrens (S1-S5) verwendet werden (S2).

11. Verfahren (S1-S5) nach einem der vorhergehenden Ansprüche, bei dem
a) eine örtliche Verteilung mindestens einer Oberflächenbeschaffenheit (BG) mehrerer Gargüter mittels des mindestens eines Sensors (11) gemessen wird,
b) aus der gemessenen Verteilung der mindestens einen Oberflächenbeschaffenheit (BG) und mehreren vorbekannten örtlichen Leistungsverteilungen (LV1, LV2) im Bereich der Gargüter ein jeweiliges Abweichungsmaß für die Gargüter gemeinsam berechnet wird, und
c) die mindestens eine Gargutbehandlungseinheit (6-9) mit dem Satz von Einstellwerten derjenigen Leistungsverteilung (LV2) betrieben wird, deren Abweichungsmaß mindestens ein vorgegebenes Kriterium erfüllt.

12. Verfahren (S1-S5) nach einem der vorhergehenden Ansprüche, bei dem
a) eine örtliche Verteilung mindestens einer Oberflächenbeschaffenheit (BG) eines Garguts mittels des mindestens eines Sensors (11) gemessen wird,
b) aus der gemessenen Verteilung der mindestens einen Oberflächenbeschaffenheit (BG) und mehreren vorbekannten örtlichen Leistungsverteilungen (LV1, LV2) im Bereich der Gargüter ein jeweiliges Abweichungsmaß für unterschiedliche Teilbereiche des Garguts mit unterschiedlichen Zielwerten der Oberflächenbeschaffenheit gemeinsam berechnet wird, und
c) die mindestens eine Gargutbehandlungseinheit (6-9) mit dem Satz von Einstellwerten derjenigen Leistungsverteilung (LV2) betrieben wird, deren Abweichungsmaß mindestens ein vorgegebenes Kriterium erfüllt.

13. Verfahren (S1-S5) nach einem der vorhergehenden Ansprüche, bei dem eine Position des Garguts (G) bestimmt wird und die vorgegebenen Leistungsverteilungen (LV1, LV2) an die bestimmte Position des Garguts (G) angepasst werden.

14. Verfahren (S1-S5) nach einem der vorhergehenden Ansprüche, bei dem eine Höhenposition des Garguts (G) an eine dazu passende Gruppe von vorgegebenen Leistungsverteilungen (LV1, LV2) anpassbar ist.

15. Haushaltsgargerät (1), aufweisend einen Garraum (2), mindestens eine Gargutbehandlungseinheit (6-9), mindestens einen Sensor (11) zum Bestimmen mindestens einer Oberflächenbeschaffenheit (BG) des Garguts (2), mindestens eine Auswerteeinrichtung (10) und eine Steuereinrichtung (10) zum Ansteuern der mindestens einer Gargutbehandlungseinheit (6-9), wobei das Haushaltsgargerät (1) dazu eingerichtet ist, das Verfahren (S1-S5) nach einem der vorhergehenden Ansprüche durchzuführen.

## Claims

1. Method (S1-S5) for treating food to be cooked (G) in a household cooking appliance (1) having a cooking compartment (2), at least one unit (6-9) for treating the food to be cooked and at least one sensor (11) for determining at least one surface condition (BG) of the food to be cooked (G) in which
a) a local distribution (LBG1, LBG2) of at least one surface condition (BG) of the food to be cooked (G) is measured by means of at least one sensor (11) (S1),
b) a respective measure of deviation is calculated from the measured dimensionless distribution (LBG1, LBG2) of the at least one surface condition (BG) and multiple dimensionless local power distributions (LV1, LV2) that are known in advance in the region of the food to be cooked (G), wherein the power distributions (LV1, LV2) that are known in advance correspond to different sets of setting values of the at least one unit (6-9) for treating the food to be cooked (S3), and
c) the at least one unit (6-9) for treating the food to be cooked is operated using the set of setting values of the power distribution (LV2) and the measure of deviation of said power distribution corresponds to the greatest deviation between the distributions (LBG1, LBG2, LV1, LV2).

2. Method (S1-S5) according to one of the preceding claims, in which the measure of deviation is a comparative summation or a difference value between the distributions.

3. Method (S1-S5) according to one of the preceding claims, in which the at least one unit (6-9) for treating the food to be cooked comprises at least one unit for treating the food to be cooked from the group having
- at least one electric radiant heating body (6-8),
- at least one induction coil,
- at least one microwave facility (9),
- at least one radiant-oriented hot air facility.

4. Method (S1-S5) according to one of the preceding claims, in which the at least one unit (6-9) for treating the food to be cooked additionally comprises at least one unit for treating the food to be cooked from the group having
- at least one radiant-oriented cooling air blower,
- at least one radiant-oriented water-supplying facility.

5. Method (S1-S5) according to one of claims 3 to 4, in which the at least one electric radiant heating body (6-8) comprises at least two radiant heating bodies (6-8), in particular from the group: bottom heating heating body (6), upper heating heating body (7) and grill heating body (8), and the set of setting values comprises setting values for at least two of the radiant heating bodies (6-8).

6. Method (S1-S5) according to claim 5, in which the power distribution (LV1, LV2) that is known in advance is only determined for one set of setting values of the at least two radiant heating bodies (6-8).

7. Method (S1-S5) according to one of the preceding claims, in which the steps a) to c) are performed cyclically.

8. Method (S1-S5) according to one of the preceding claims, in which the at least one sensor (11) comprises at least one optical sensor and/or at least one infrared sensor.

9. Method (S1-S5) according to one of the preceding claims, in which the at least one surface condition comprises a degree of browning, a surface temperature and/or a moisture.

10. Method (S1-S5) according to one of the preceding claims, in which a position (E) of the food to be cooked (G) in the cooking compartment (2) is determined and only the local power distributions (LV1, LV2) and distributions (LBG1, LBG2) of the surface condition (BG) that relate to this position are used in order to implement the method (S1-S5) (S2).

11. Method (S1-S5) according to one of the preceding claims, in which
a) a local distribution of at least one surface condition (BG) of multiple items of food to be cooked is measured by means of the at least one sensor (11),
b) a respective measure of deviation for the items of food to be cooked is calculated jointly from the measured distribution of the at least one surface condition (BG) and multiple local power distributions (LV1, LV2) that are known in advance in the region of the items of food to be cooked, and
c) the at least one unit (6-9) for treating the food to be cooked is operated using the set of setting values of the power distribution (LV2) and the measure of deviation of said power distribution fulfils at least one predetermined criterion.

12. Method (S1-S5) according to one of the preceding claims, in which
a) a local distribution of at least one surface condition (BG) of one item of food to be cooked is measured by means of the at least one sensor (11),
b) a respective measure of deviation for different part regions of the food to be cooked having different target values of the surface condition is calculated jointly from the measured distribution of the at least one surface condition (BG) and multiple local power distributions (LV1, LV2) that are known in advance in the region of the items of food to be cooked, and
c) the at least one unit (6-9) for treating the food to be cooked is operated using the set of setting values of the power distribution (LV2) and the measure of deviation of said power distribution fulfils at least one predetermined criterion.

13. Method (S1-S5) according to one of the preceding claims, in which a position of the food to be cooked (G) is determined and the predetermined power distributions (LV1, LV2) are adapted to the determined position of the food to be cooked (G).

14. Method (S1-S5) according to one of the preceding claims, in which a height position of the food to be cooked (G) can be adapted to a group of predetermined power distributions (LV1, LV2) that is suitable for this height.

15. Household cooking appliance (1), having a cooking compartment (2), at least one unit (6-9) for treating the food to be cooked, at least one sensor (11) for determining at least one surface condition (BG) of the food to be cooked (2), at least one evaluating facility (10) and a control facility (10) for controlling the at least one unit (6-9) for treating the food to be cooked, wherein the household cooking appliance (1) is configured for the purpose of implementing the method (S1-S5) according to one of the preceding claims.

## Revendications

1. Procédé (S1-S5) de traitement d'un aliment à cuire (G) dans un appareil de cuisson domestique (1) comprenant une chambre de cuisson (2), au moins une unité de traitement d'aliment à cuire (6-9) et au moins un capteur (11) pour déterminer au moins une caractéristique de surface (BG) de l'aliment à cuire (G), dans lequel :
a) une répartition locale (LBG1, LBG2) d'au moins une caractéristique de surface (BG) de l'aliment à cuire (G) est mesurée (S1) au moyen d'au moins un capteur (11),
b) une grandeur d'écart respective est calculée à partir de la répartition, sans dimension, mesurée (LBG1, LBG2) de l'au moins une caractéristique de surface (BG) et de plusieurs répartitions de puissance (LV1, LV2) locales sans dimension préalablement connues dans la zone de l'aliment à cuire (G), dans lequel les répartitions de puissance (LV1, LV2) préalablement connues correspondent (S3) à des ensembles différents de valeurs de consigne d'au moins une unité de traitement d'aliment à cuire (6-9), et
c) l'au moins une unité de traitement d'aliment à cuire (6-9) est commandée avec l'ensemble de valeurs de consigne de la répartition de puissance (LV2) dont la grandeur d'écart correspond au plus grand écart entre les répartitions (LBG1, LBG2, LV1, LV2).

2. Procédé (S1-S5) selon l'une des revendications précédentes, dans lequel la grandeur d'écart est une somme de comparaison ou une somme de différences entre les répartitions.

3. Procédé (S1-S5) selon l'une des revendications précédentes, dans lequel l'au moins une unité de traitement d'aliment à cuire (6-9) comprend au moins une unité de traitement d'aliment à cuire parmi le groupe comprenant les suivants :
- au moins un corps de chauffe électrique à rayonnement (6-8),
- au moins une bobine à induction,
- au moins un dispositif à micro-ondes (9),
- au moins un dispositif d'air chaud dirigé par un jet.

4. Procédé (S1-S5) selon l'une des revendications précédentes, dans lequel l'au moins une unité de traitement d'aliment à cuire (6-9) comprend en outre au moins une unité de traitement d'aliment à cuire parmi le groupe comprenant les suivants :
- au moins un ventilateur d'air de refroidissement dirigé par un jet,
- au moins un dispositif d'alimentation d'eau dirigé par un jet.

5. Procédé (S1-S5) selon l'une des revendications 3 à 4, dans lequel l'au moins un corps de chauffe électrique à rayonnement (6-8) comprend au moins deux corps de chauffe électriques à rayonnement (6-8), en particulier parmi le groupe comprenant des corps de chauffe à chaleur de sole (6), des corps de chauffe à chaleur de voûte (7) et des corps de chauffe de grill (8), et les ensembles de valeurs de consigne comprennent des valeurs de consigne pour au moins deux des corps de chauffe à rayonnement (6-8).

6. Procédé (S1-S5) selon la revendication 5, dans lequel la répartition de puissance préalablement connue (LV1, LV2) est déterminée uniquement pour un ensemble de valeurs de consigne des au moins deux corps de chauffe à rayonnement (6-8).

7. Procédé (S1-S5) selon l'une des revendications précédentes, dans lequel les opérations a) à c) sont exécutées de façon cyclique.

8. Procédé (S1-S5) selon l'une des revendications précédentes, dans lequel l'au moins un capteur (11) comprend au moins un capteur optique et/ou au moins un capteur infrarouge.

9. Procédé (S1-S5) selon l'une des revendications précédentes, dans lequel l'au moins une caractéristique de surface comprend un degré de brunissement, une température de surface et/ou une humidité.

10. Procédé (S1-S5) selon l'une des revendications précédentes, dans lequel une position (E) de l'aliment à cuire (G) dans la chambre de cuisson (2) est déterminée et seules les répartitions de puissance (LV1, LV2) locales et les répartitions (LBG1, LBG2) de la caractéristique de surface (BG) se rapportant à cette position sont utilisées pour exécuter le procédé (S1-S5).

11. Procédé (S1-S5) selon l'une des revendications précédentes, dans lequel
a) une répartition locale d'au moins une caractéristique de surface (BG) de plusieurs aliments à cuire (G) est mesurée (S1) au moyen de l'au moins un capteur (11),
b) une grandeur d'écart respective est calculée en commun pour les aliments à cuire à partir de la répartition mesurée de l'au moins une caractéristique de surface (BG) et de plusieurs répartitions de puissance (LV1, LV2) locales préalablement connues dans la zone des aliments à cuire, et
c) l'au moins une unité de traitement d'aliment à cuire (6-9) est commandée avec l'ensemble de valeurs de consigne de la répartition de puissance (LV2) dont la grandeur d'écart satisfait à au moins un critère prédéfini.

12. Procédé (S1-S5) selon l'une des revendications précédentes, dans lequel
a) une répartition locale d'au moins une caractéristique de surface (BG) d'un aliment à cuire est mesurée (S1) au moyen de l'au moins un capteur (11),
b) une grandeur d'écart respective est calculée en commun pour différentes zones partielles de l'aliment à cuire ayant différentes valeurs cibles de la caractéristique de surface, à partir de la répartition mesurée de l'au moins une caractéristique de surface (BG) et de plusieurs répartitions de puissance (LV1, LV2) locales préalablement connues dans la zone des aliments à cuire, et
c) l'au moins une unité de traitement d'aliment à cuire (6-9) est commandée avec l'ensemble de valeurs de consigne de la répartition de puissance (LV2) dont la grandeur d'écart satisfait à au moins un critère prédéfini.

13. Procédé (S1-S5) selon l'une des revendications précédentes, dans lequel une position de l'aliment à cuire (G) est déterminée et les répartitions de puissance prédéfinies (LV1, LV2) sont adaptées à la position déterminée de l'aliment à cuire (G).

14. Procédé (S1-S5) selon l'une des revendications précédentes, dans lequel une position en hauteur de l'aliment à cuire (G) peut être adaptée à un groupe de répartitions de puissance prédéfinies (LV1, LV2) approprié.

15. Appareil de cuisson domestique (1), comprenant une chambre de cuisson (2), au moins une unité de traitement d'aliment à cuire (6-9), au moins un capteur (11) pour déterminer au moins une caractéristique de surface (BG) de l'aliment à cuire (2), au moins un moyen d'évaluation (10) et un dispositif de commande (10) pour commander au moins une unité de traitement d'aliment à cuire (6-9), dans lequel l'appareil de cuisson domestique (1) est configuré pour exécuter le procédé (S1-S5) selon l'une des revendications précédentes.
